# EUROPEAN PATENT SPECIFICATION

(11) **EP 3 765 056 B1**
(45) Date of publication and mention of the grant of the patent: **02.08.2023**
(21) Application number: 19712494.4
(22) Date of filing: 13.03.2019
(51) Int. Cl.: A61K 38/17, A61P 25/22, A61P 25/36

(54) **COMPOSITIONS, METHODS AND USES OF A TENEURIN C-TERMINAL ASSOCIATED PEPTIDE-1 (TCAP-1) FOR TREATING OPIOID ADDICTION**
ZUSAMMENSETZUNGEN, VERFAHREN UND VERWENDUNGEN EINES TENEURIN-C-TERMINALEN ASSOZIIERTEN PEPTID-1 (TCAP-1) ZUR BEHANDLUNG VON OPIOIDSUCHT
COMPOSITIONS, MÉTHODES ET UTILISATIONS D'UN PEPTIDE-1 ASSOCIÉ À L'EXTRÉMITÉ C-TERMINALE DE LA TÉNEURINE (TCAP-1) POUR TRAITER LA DÉPENDANCE AUX OPIOÏDES

(30) Priority: 13.03.2018 US 201862642201 P; 12.10.2018 WO PCT/US2018/055732
(43) Date of publication of application: 20.01.2021
(73) Proprietor: Protagenic Therapeutics, Inc., New York NY 10010 (US)
(72) Inventor: SLEE, Andrew, Shrewsbury, MA 01545 (US); STEIN, Robert, New York, NY 10010 (US); ARMEN, Garo, New York, NY 10010 (US)
(74) Representative: D Young & Co LLP
(86) International application number: PCT/US2019/022060
(87) International publication number: WO 2019/178234

(56) References cited:
- US-A1- 2006 035 318
- US-A1- 2008 318 854
- LIQUN WANG ET AL: "Teneurin proteins possess a carboxy terminal sequence with neuromodulatory activity", MOLECULAR BRAIN RESEARCH, vol. 133, no. 2, 1 February 2005 (2005-02-01), pages 253-265, XP055540792, NL ISSN: 0169-328X, DOI: 10.1016/j.molbrainres.2004.10.019
- LAURA A. TAN ET AL: "Repeated intracerebral teneurin C-terminal associated peptide (TCAP)-1 injections produce enduring changes in behavioral responses to corticotropin-releasing factor (CRF) in rat models of anxiety", BEHAVIOURAL BRAIN RESEARCH., vol. 188, no. 1, 1 March 2008 (2008-03-01) , pages 195-200, XP055540793, NL ISSN: 0166-4328, DOI: 10.1016/j.bbr.2007.10.032
- LAURA A. TAN ET AL: "Teneurin C-terminal associated peptide (TCAP)-1 modulates dendritic morphology in hippocampal neurons and decreases anxiety-like behaviors in rats", PHYSIOLOGY AND BEHAVIOR, vol. 104, no. 2, 1 August 2011 (2011-08-01), pages 199-204, XP055540795, GB ISSN: 0031-9384, DOI: 10.1016/j.physbeh.2011.03.015
- REBECCA WOELFLE ET AL: "Teneurins, TCAP, and latrophilins: roles in the etiology of mood disorders", TRANSLATIONAL NEUROSCIENCE, vol. 7, no. 1, 1 January 2016 (2016-01-01) , pages 17-23, XP055540802, Heidelberg ISSN: 2081-3856, DOI: 10.1515/tnsci-2016-0004
- TAN L A ET AL: "Teneurin C-terminal associated peptide (TCAP)-1 attenuates corticotropin-releasing factor (CRF)-induced c-Fos expression in the limbic system and modulates anxiety behavior in male Wistar rats", BEHAVIOURAL BRAIN RESEARCH, ELSEVIER, AMSTERDAM, NL, vol. 201, no. 1, 19 July 2009 (2009-07-19) , pages 198-206, XP026020931, ISSN: 0166-4328, DOI: 10.1016/J.BBR.2009.02.013 [retrieved on 2009-02-21] cited in the application
- AL CHAWAF ET AL: "Corticotropin-releasing factor (CRF)-induced behaviors are modulated by intravenous administration of teneurin C-terminal associated peptide-1 (TCAP-1)", PEPTIDES, ELSEVIER, AMSTERDAM, NL, vol. 28, no. 7, 26 July 2007 (2007-07-26), pages 1406-1415, XP022168929, ISSN: 0196-9781, DOI: 10.1016/J.PEPTIDES.2007.05.014
- DAVID A. KUPFERSCHMIDT ET AL: "Teneurin C-terminal associated peptide-1 blocks the effects of corticotropin-releasing factor on reinstatement of cocaine seeking and on cocaine-induced behavioural sensitization : TCAP-1 blocks effects of CRF on cocaine-related behaviours", BRITISH JOURNAL OF PHARMACOLOGY, vol. 162, no. 3, 12 January 2011 (2011-01-12), pages 574-583, XP55584101, UK ISSN: 0007-1188, DOI: 10.1111/j.1476-5381.2010.01055.x
- REBECCA WOELFLE ET AL: "Ancient interaction between the teneurin C-terminal associated peptides (TCAP) and latrophilin ligand-receptor coupling: a role in behavior", FRONTIERS IN NEUROSCIENCE, vol. 9, 24 April 2015 (2015-04-24), XP55584108, DOI: 10.3389/fnins.2015.00146

## Description

### FIELD

The present invention relates to compositions of a Teneurin C-Terminal Associated Peptide - 1 (TCAP-1) for use in preventing and/or treating opioid addiction.

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### BACKGROUND

Opioids act by binding to opioid receptors, which are found principally in the central and peripheral nervous system and the gastrointestinal tract. These receptors mediate both the psychoactive and the somatic effects of opioids. Opioids are often prescribed for pain relief (or to alter the perception of pain) especially after a trauma (e.g. back or other injuries or surgery). However, they also have a considerably high addiction rate and withdrawal symptoms make it more difficult to overcome same.

Opioid drugs include partial agonists, like the anti-diarrhea drug loperamide and antagonists like naloxegol for opioid-induced constipation, which do not cross the blood-brain barrier, but can displace other opioids from binding in those receptors.

Because of opioid drugs' reputation for addiction and fatal overdose, most are controlled substances. In 2013, between 28 and 38 million people used opioids illicitly (0.6% to 0.8% of the global population between the ages of 15 and 65). In 2011, an estimated 4 million people in the United States used opioids recreationally or were dependent on them. As of 2015, increased rates of recreational use and addiction are attributed to over-prescription of opioid medications and inexpensive illicit heroin. Conversely, fears about over-prescribing, exaggerated side effects and addiction from opioids are similarly blamed for under-treatment of pain.

Opioid addiction is different from other addictions such as cocaine. Unlike opioids such as morphine which is a pain reliever and tends to make one more relaxed, cocaine acts by inhibiting the reuptake of serotonin, norepinephrine, and dopamine. This results in greater concentrations of these three neurotransmitters in the brain and energy increase. It can easily cross the blood-brain barrier and may lead to the breakdown of the barrier. Further, morphine impacts the body differently in the reward system of the brain. Research investigating exactly how and why opioids cause addition is important to learning how to combat the situation effectively.

There is a need for preventing and treating opioid addiction.

### SUMMARY OF THE INVENTION

The present invention provides a teneurin c-terminal associated peptide-1 (TCAP-1 peptide) and compositions comprising same for use in preventing and/or treating opioid addiction and associated conditions or symptoms, such as withdrawal.

In one aspect, the invention provides a teneurin c-terminal associated peptide-1 (TCAP-1 peptide), in a therapeutically effective amount, or a pharmaceutically acceptable salt or ester thereof or a pharmaceutical composition comprising same for use in preventing and/or treating opioid addiction in a subject in need thereof, wherein the amino acid sequence of said TCAP-1 peptide consists essentially of:
(i) an amino acid sequence having at least 95% identity to an amino acid sequence selected from the group consisting of SEQ ID NOs: 1, 2 or 3;
optionally wherein:
(a) the carboxy terminal end of said TCAP peptide is amidated or comprises an amidation signal sequence; and/or
(b) when the amino terminal amino acid of said TCAP peptide is glutamine, it is in the form of pyroglutamic acid.

In other aspects, a TCAP-1 peptide can be administered prior to, contemporaneous with and/or after opioid use.

In some embodiments the patient or subject is a mammal. In some other embodiments, the mammal is selected from the group consisting of humans, dogs, cats, horses, sheep and cattle. In some embodiments the patient or subject is human.

### BRIEF DESCRIPTION OF THE DRAWINGS

In order that the subject matter may be readily understood, embodiments are illustrated by way of examples in the accompanying drawings, in which:
**Figure 1** is the TCAP-1 SEQ. ID. Nos. 1 - 3 for mouse, human and G.Gallus, respectively.
**Figure 2** is a schematic illustration of the study design of Example 1 for the mouse Saelens drug-precipitated withdrawal study where TCAP-1 was administered pre-morphine.
**Figure 3** is a table illustrating the results of Example 1 showing that TCAP-1 blunts opioid withdrawal stress response in a dose dependent manner.
**Figure 4** is a bar graph illustrating the results of Figure 3 that TCAP-1 blunts opioid withdrawal stress response in a dose dependent manner.
**Figure 5** is a schematic illustration of the saelens test in mice - single dose TCAP-1 post-morphine as described in Example 2.
**Figure 6** is a table illustrating the results of Example 2.
**Figure 7** is a bar graph illustrating the results of Figure 6.
**Figure 8A** is a schematic illustration of the study design of Example 3.
**Figure 8B** is a bar graph showing the results of the precipitated withdrawal test in mice of Example 3, specifically # jumps/20 minutes in mice versus vehicle used (saline, morphine alone, CRH antagonist (CP154,526) and TCAP-1.
**Figure 9A** is a bar graph showing results of Saelens precipitated withdrawal test in mice of Example 3 of Group Mean (# jumps/20 minutes) versus noted vehicle (saline, CP154,526 (5, 20, and 50 mg/kg) and TCAP-1 250nmole/kg) in a dose response study.
**Figure 9B** is a bar group of plasma corticosterone levels in mice using the Saelens precipitated withdrawal model of Example 3 in the dose response study, showing Group mean ng/ml of plasma corticosterone versus vehicle (saline, CP154,526 (5,20, and 50 mg/kg) and TCAP-1 250nmole/kg).
**Figure 10** is a schematic illustration of the study design of Example 4.
**Figure 11** is a bar graph illustrating the Saelens precipitated withdrawal test in mouse of Example 4 showing #jumps/t-5-20 minutes versus vehicle (no treatment (F>N), TCAP-1 250 nmole/kg and 500 nmole/kg in fentanyl treated mice.

### DETAILED DESCRIPTION

Teneurin C-terminal associated peptides (TCAPs 1-4) are four paralogous bioactive peptides located at the distal extracellular end of each teneurin transmembrane protein. First described by Lovejoy et al and described in US Patent No. 8,088,889. TCAP-1 can be independently (and synthetically) transcribed and has biological actions distinct from the teneurins, demonstrating functional independence from its proprotein. It has a unique mechanism of action. ADGRL (Latrophilin), an adhesion G protein-coupled receptor (GPCR), has recently been identified as part of the ligand-receptor complex that binds the teneurin/TCAP system. Previously elucidated in neurons, the teneurin/TCAP-ADGRL complex is associated with glucose metabolism; however, it is not well understood in other tissues.

Opioid addiction has been linked with corticotrophin releasing hormone (CRH) driven behaviours, such as jumping, grooming, and inhibition of appetite.

### DEFINITIONS

**"Preventing"** as used herein in reference to preventing opioid addiction or onset means in the context of administering a TCAP-1 to a patient who is or will be or may be receiving opioids to minimize risk of developing an addiction to opioids and includes preventing or minimizing risk of relapse or recidivism.

**"Stressor" or "Traumatic Event"** as used herein means an event or events that precipitates the use of or an addiction to an opioid, e.g., any event that may be painful, e.g. surgery, injury, physiological or hormonal (e.g. fibroids, menses) or behavioural (e.g. a scare, remembering stress events).

**"Therapeutically Effective Amoun**t**"** as used herein when applied to dose or amount refers to that quantity of a compound or pharmaceutical composition that is sufficient to result in a desired activity upon administration to a living animal body. It is understood that a therapeutic amount may vary depending on a number of factors, including but not limited to gender, weight, body mass or body surface area, severity of a condition, age (e.g., child, teen, adult, or senior).
**"Treating" or "treatment"** as used herein in the context of opioid addiction means alleviating or controlling or managing the symptoms of opioid addiction and/or withdrawal and not necessarily "curing" or "curing permanently" opioid addiction but can also include same.

### DESCRIPTION

Opioid addiction is a major unmet medical need. Current treatments for opioid addiction are suboptimal with regard to efficacy, side effects, or both. Corticotrophin releasing hormone (CRH), a hypothalamic neuropeptide, plays a central role in mental and behavioral responses to environmental stress. CRH is overproduced in opioid withdrawal patients, and they seem to be more sensitive to the actions of CRH.

Although TCAP and teneurins have been largely studied in the brain and shown to have normalizing effects on stressed or high and low anxiety animals (See US Patent No. 8,088,889). The TCAP, portion of teneurin proteins, blocks CRH activities through a separate receptor called latrophilin and synthesized TCAP given to rodents reverses stress-induced behavioral dysfunction, including anxiety and depression. It has better relief of illness in current responders and greater efficacy in current non-responders with fewer side effects, e.g. less sedation, less harmful pharmacology and less dependency.

TCAP-1 calms anxious behavior without sedation or evidence of dependency. As an intervention in drug addiction, in one aspect TCAP-1 can be used to reduce drug seeking behavior. In another aspect, it could be used without interfering with reward circuitry to depress mood. The pharmacological benefits of TCAP-1 administration persist for significant periods after one dose, reflecting its ability to restore long-term balance to brain function. It appears to counteract CRH effects.

Prior to the present invention, no studies were previously done in an opioid addiction model/protocol which is a separate and distinct addiction. Therefore, the inventors examined the specific role of TCAP-1 in such a model. As illustrated herein it has been shown that a TCAP-1 peptide could be used to treat and/or prevent opioid addiction and that it counteracts CRH effects and is more effective than a CRH antagonist.

### Teneurin C-Terminal Associated Peptide-1 (TCAP-1)

TCAP-1 as used herein is a peptide that consists of a sequence found at the c-terminal of Teneurin M-1 peptide, more particularly described below (and collectively referred herein as "a TCAP-1 peptide"). There is considerable cross-species homology.

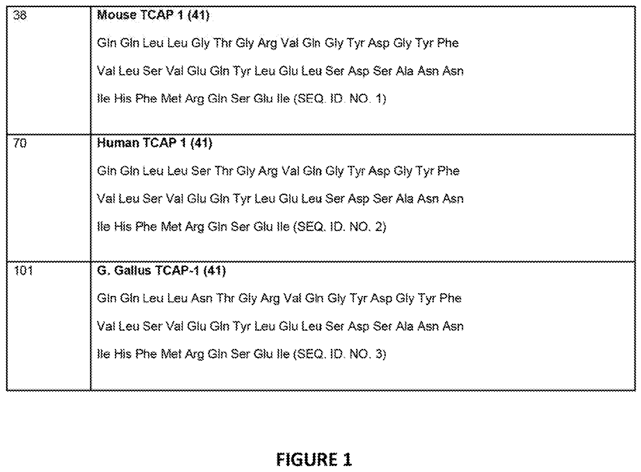

In some embodiments the TCAP-1 peptide ("TCAP-1") is a 41-mer peptide selected from SEQ. ID. NOs 1 to 3 (see also **Figure 1**). In some embodiments it is an amidated peptide, (such as a C-terminal amidated peptide and/or having an amidation signal sequence, such as GRR), in some other embodiments the TCAP has a pyroglutamic acid at the N-terminal. In other embodiments, it has both a pyroglutamic acid at the N-terminal and is amidated at the C-terminal.

In other embodiments it is a human TCAP-1. In some embodiments it is a 41-mer c-terminal amidated peptide consisting of the following sequence:
Amidated Human TCAP-1 (41 mer) 
Gln* Gln Leu Leu Ser Thr Gly Arg Val Gln Gly Tyr Asp Gly Tyr Phe
Val Leu Ser Val Glu Gln Tyr Leu Glu Leu Ser Asp Ser Ala Asn Asn
Ile His Phe Met Arg Gln Ser Glu Ile – NH2
* In some embodiments the N-terminal glutamic acid may be a pyroglutamic acid.

In some other embodiments, the peptide used is a salt, ester, solvate, polymorph or enantiomer of SEQ. ID. NOs. 1 to 3, preferably SEQ. ID. NO. 1, or any amidated or pyroglutamic acid or amidated and pyroglutamic acid form of SEQ. ID. NOs. 1 to 3.

In some other embodiments, conservative substitutions or modifications can be made to the peptide sequence which does not affect its structure or function and thus could be used for the present invention, such as various species homologs. For instance, those present in species homologs, such as the mouse, human or G. gallus TCAP-1 sequences (SEQ. ID. NOs. 1 - 3) where the fifth amino acid may be selected from: Gly, Asn or Ser. In some embodiments, the peptide has 95% identity to SEQ. ID. NOs. 1, 2, or 3. There is a high degree of homology amongst species, for instance the mouse TCAP-1 (Mus musculus) has the same sequence as the rat TCAP-1 (Rattus norvegicus), while the human TCAP-1 and that of the long-tailed Macaque (Macaca fascicularis) are the same.

As described herein, the TCAP-1 peptide comprises any one of SEQ. ID. NOs. 4 -9 or a salt, ester, solvate, polymorph or enantiomer of thereof, or any amidated or pyroglutamic acid, or amidated and pyroglutamic acid form thereof.

### Opioids

Opiates are alkaloids originally derived from the poppy plant. People use this type of drug for both recreational and medicinal purposes. There are opiates that come from the natural opium plant, while some manufacture opiates to have the same chemical structure as the natural ones, e.g. synthetic or semi-synthetic opioids. Furthermore, opiates include a wide range from prescription painkillers such as fentanyl and morphine, to illegal drugs like heroin.

Natural opioids come from natural sources such as the opium plant. While some labs completely manufacture opioid drugs, natural ones come directly from the poppy seed. Although some think natural opiates are less risky than synthetic ones, they are still very addictive and can cause respiratory depression that leads to an overdose.

Synthetic opiates act on the same areas of the brain as natural ones and produce many of the same effects. They are entirely human-made with chemicals not found in the poppy plant, morphine or opium. Thus, the chemicals used in these synthetic drugs vary.

Semi-synthetic opiates are a blend of natural and man-made sources developed.

Examples of opioids include but are not limited to: Opium, Heroin, Oxycodone (e.g.,Oxycontin, Roxicodone, Xtampza ER, and Oxaydo), Hydrocodone (e.g., Vicodin, Lorcet, Lortab), Codeine, Morphine (MS Contin, Oramorph SR, Avinza, and Arymo ER), Hydromorphone (Dilaudid and Exalgo), Fentanyl (Actiq, Fentora, Duragesic, Subsys, Abstral, and Lazanda), Methadone (Dolophine and Methadose), Meperidine (Demerol); Opioid analgesic: Tramadol (ConZip, Ultram, and Ryzolt); Carfentanil .

### Opioid Withdrawal Symptoms

Withdrawal symptoms range from mild to severe. Indeed, symptoms can vary based on the length of time taking a particular drug, dosage, which specific drug, method of use, medical conditions, the presence of emotional issues, and biological and environmental factors. In general, opiate withdrawal usually starts within 6 to 12 hours for short-acting opiates, and within 30 hours for longer-acting ones. Symptoms may include but are not limited to: muscle aches, trouble falling and staying asleep, yawning, anxiety, runny nose, sweating, rapid heart rate, hypertension, nausea and vomiting, and diarrhea.

There are well known accepted animal models and tests that have been developed and used to screen for opiate withdrawal effects, such as analgesic responses to opiates and other agents. The mouse jumping test is a simple screening method to estimate the physical dependence capacity of analgesics. (See for example, Saelens JK, Granat FR, Sawyer WK, The mouse jumping test--a simple screening method to estimate the physical dependence capacity of analgesics. Arch Int Pharmacodyn Ther. 1971. Apr;190(2):213-8 ; Rainer Spanagel, PhD*, Animal models of addiction, Dialogues Clin. Neurosci. 2017 Sep;19(3):247-258; Wendy J Lynch, Katherine L Nicholson, Mario E Dance, Richard W Morgan, Patricia L Foley, Animal Models of Substance Abuse and Addiction: Implications for Science, Animal Welfare, and Society, Comp Med. 2010 Jun; 60(3): 177-188.)

### Pharmaceutical Compositions

In one embodiment the present invention relates to a pharmaceutical composition comprising a teneurin c-terminal associated peptide-1 (TCAP-1 peptide) as described in claim 1 for use in preventing and/or treating opioid addiction in a subject in need thereof. The use comprises the administration of a pharmaceutical composition comprising a TCAP-1 peptide as described in claim 1 (including an amidated and/or pyroglutamic acid form of TCAP-1 or a peptide with 95% identity to SEQ. ID. NOs. 1- 3 as shown in Figure 1) and a pharmaceutically acceptable carrier.

The phrase **"pharmaceutically acceptable**", as used in connection with compositions of the invention, refers to molecular entities and other ingredients of such compositions that are physiologically tolerable and do not typically produce untoward reactions when administered to a mammal (e.g., human). Preferably, as used herein, the term "**pharmaceutically acceptable**" means approved by a regulatory agency of the Federal or a state government or listed in the U.S. Pharmacopeia (USP), National Formulary (NF), or other generally recognized pharmacopeia for use in mammals, and more particularly in humans. Active Pharmaceutical Ingredients (APIs) of the present invention may be in the form of pharmaceutically acceptable salts. "**Pharmaceutically acceptable salts**" refers to those salts which possess the biological effectiveness and properties of the parent compound and which are not biologically or otherwise undesirable.

The pharmaceutical compositions for use of the present invention may comprise one or more excipients. Excipients which may be used include carriers, surface active agents (surfactants), thickening (viscosity) agents, emulsifying agents, binding agents, dispersion or suspension agents, buffering agents, penetration-enhancing agents, solubilizers, colorants, sweeteners, flavoring agents, coatings, disintegrating agents, lubricants, preservatives, isotonic agents, and combinations thereof. The selection and use of suitable excipients is taught in Gennaro, ed., Remington: The Science and Practice of Pharmacy, 20th Ed. (Lippincott Williams & Wilkins 2003).

The term "**carrier**" applied to pharmaceutical compositions for use of the invention refers to a diluent, excipient, or vehicle with which an active compound is administered. Such pharmaceutical carriers can be liquids, such as water, saline solutions, aqueous dextrose solutions, aqueous glycerol solutions, and lipids and oils, including those of petroleum, animal, vegetable or synthetic origin. Suitable pharmaceutical carriers are described in "Remington's Pharmaceutical Sciences" by E. W. Martin, 18.sup.th Edition.

In some embodiments, the dosage form is a subcutaneous dosage form. This differs from direct administration to the brain, amygdala, or Intracerebroventricular ("ICV"). Subcutaneous administration has many advantages over direct administration to the brain.

In some embodiments as in the composition used in the Examples, the composition dissolves an amidated and/or pyroglutamic acid form of TCAP in a saline solution and is subcutaneously administered into animals (not ICV or amygdala). This formulation has advantages over prior forms for delivery, i.e., ICV or amygdala, in that it does not require additional sedatives, or the like for administration. In other embodiments, the formulation is an oral (buccal or sublingual) or nasal formulation.

The compositions may, if desired, be presented in a pack or dispenser device which may contain one or more unit dosage forms containing the active ingredient(s). The pack may, for example, comprise metal or plastic foil, such as a blister pack. Compositions for use of the invention formulated in a compatible pharmaceutical carrier may also be prepared, placed in an appropriate container, and labeled for treatment of an indicated condition.

TCAP-1 and the pharmaceutical compositions of the invention are for use in preventing or treating opioid addiction in an animal, in some embodiments mammals, including but not limited to humans, dogs, cats, horses, sheep, cattle.

### METHODS AND USES

A TCAP-1 peptide as described herein and the pharmaceutical compositions comprising are for use in preventing and/or treating opioid addiction, and/or assist to minimize withdrawal symptoms of opioid addiction. In some embodiments it can be used or administered to a patient before e.g., an anticipated use of opioids, such as for instance surgery, injury and/or during or after a traumatic event. In some embodiments, the traumatic event is selected from the group consisting of: a medical procedure such as surgery, cramping, including but not limited to associated with fibroids or menses, back or joint pain, injury, or inflammation, or other event.

In some embodiments the TCAP-1 peptide and pharmaceutical compositions comprising same are for use in methods for treating and/or preventing opioid addiction or associated withdrawal symptoms by administering an effective amount of a TCAP-1 peptide to a patient in need thereof, before, during or after a traumatic event or stressor or anticipated traumatic event or stressor where opioid use and ceasing of such use is anticipated. For instance, when a person undergoes surgery, opioids to control the pain may be prescribed for a set period of time. A TCAP-1 peptide may be used to assist a patient when said prescription ends to reduce addiction or risk of addiction and assist in transitioning the patient off opioid use.

In some other embodiments, a TCAP-1 peptide can be used in a opioid addiction treatment and/or prevention protocol.

The present invention is described in the following Examples, which are set forth to aid in the understanding of the invention, and should not be construed to limit in any way the scope of the invention as defined in the claims which follow thereafter.

### EXAMPLES

The following examples illustrate the role of TCAP-1 in preventing and treating opioid addiction.

### MATERIALS AND METHODS

### TCAP-1 Composition

Amidated human TCAP-1 (SEQ. ID. NO. 1) was suspended in 0.9% saline. [10 nmol/Kg, Ambiopharm] for subcutaneous injection in the interscapular region.

Amidated human TCAP-1 peptide used in the composition was synthesized on an automated peptide synthesizer, Model Novayn Crystal (NovaBiochem) on PEG-PS resin using continuous flow Fmoc chemistry (Calbiochem-NovabiochemGroup). Eight times excess diisopropyl ethyl amine (Sigma-Aldrich) and four times excess Fmoc-amino acid activated with HATU (O-(7-azabenzotriazol)-1-3, 3-tetramethyluronium hexfluorophosphate; Applied Biosystems) at a 1:1 (mol/mol) ratio were used during the coupling reaction. The reaction time was 1 h. A solution of 20% piperidine (Sigma-Aldrich) in N, N-dimethylformide (DMF; Caledon Laboratories) was used for the deprotection step in the synthesis cycle. The DMF was purified inhouse and used fresh each time as a solvent for the synthesis. The cleavage/deprotection of the final peptide was carried out with trifluoroacetic acid (TFA), thioanisole, 1, 2 ethandithiol, m-cresole, triisopropylsilane, and bromotrimethyl silane (Sigma-Aldrich) at a ratio of 0:10:5:1:1:5. Finally, it was desalted on a Sephadex G-10 column using aqueous 0.1% TFA solution and lyophilized.

In some experiments mTCAP was also used and prepared similarly to hTCAP-1 as above.

### Animals

All animal studies were performed in the United States and followed the requirements set out in applicable laws, including the Animal Health and Welfare Act and regulations and approved by the Institutional Animal Care and Use Committee of Neosome Life Sciences, Lexington MA USA.

Male mice Swiss Webster mice from Charles River Laboratory (18 - 22 g). All procedures were approved by Neosome Life Sciences IACUC and in accordance with applicable laws and guidelines regarding use of animals.

### Statistics

Tests were used to assess statistical significances. Student's t-test and ANOVAs were used unless specifically stated otherwise. Statistics were denoted by *p<0.05, **p<0.01, ***p<0.001, ****p<0.0001.

### CLINICAL TRIALS IN AN OPIOID ADDICTION MODEL

### EXAMPLE 1: Saelens Drug-Precipitated Withdrawal In the Mouse - TCAP-1 Pre-Morphine

### Protocol

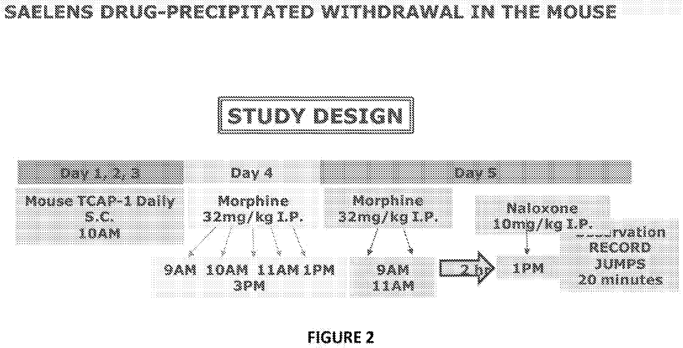

A schematic of the protocol can be seen in **Figure 2****.**

Mice were administered saline (Groups 1, 2 and 8) or TCAP-1 (Groups 2-7) solution daily subcutaneously for three days at 10 a.m. each day.

On day four the mice were administered saline (Groups 1 and 8) or 32 mg/kg of morphine by intraperitoneal injection (I.P.) (Groups 2 - 7) at 9, 10, 11 a.m. and 1 and 3 p.m.

On day five, the mice were administered saline (Groups 1), 32 mg/kg of morphine by intraperitoneal injection (I.P.) (Groups 2 - 7) or MK-801 (Group 8) at 9 and 11 a.m.

Two hours after the 11 a.m. injection (at 1 p.m.), naloxone 10 mg/kg I.P was injected and the mice were observed over 20 minutes and number of jumps recorded.

Naloxone is a non-selective and competitive opioid receptor antagonist. It works by reversing the depression of the central nervous system and respiratory system caused by opioids. It is a medication used to block the effects of opioids, especially in overdose. Administration to opioid-dependent individuals may cause symptoms of opioid withdrawal, including restlessness, agitation, nausea, vomiting, a fast heart rate, and sweating. It induces withdrawal.

MK-801 is a non-competitive antagonist of N-Methyl D aspartate receptor, a glutamate receptor which is an excitatory neurotransmitter.

**TABLE 1**

| **Group** | **Days 1, 2, and 3 (10 a.m.)** | **Day 4 (9 a.m., 10 a.m., 11 a.m., 1 p.m. and 3 p.m.) and Day 5 9 and 11 a.m.)** | **Day 5 (1 p.m.)** | **Mean # jumps over 20 minutes** |
|---|---|---|---|---|
| 1 n=9 | Saline | Saline | Saline | 1 |
| 2 n=9 | Saline | Morphine 32 mg/kg i.p. | Naloxone 10mg/kg i.p. | 73 |
| 3 n=9 | TCAP-1 10 nmole/kg | Morphine 32 mg/kg i.p. | Naloxone 10mg/kg i.p. | 47 |
| | | | | |
| 4 n=9 | TCAP-1 25 nmole/kg | Morphine 32 mg/kg i.p. | Naloxone 10mg/kg i.p. | 47 |
| 5 n=9 | TCAP-1 50 nmole/kg | Morphine 32 mg/kg i.p. | Naloxone 10mg/kg i.p. | 42 |
| 6 n=9 | TCAP-1 100 nmole/kg | Morphine 32 mg/kg i.p. | Naloxone 10mg/kg i.p. | 32 |
| 7 n=9 | TCAP-1 250 nmole/kg | Morphine 32 mg/kg i.p. | Naloxone 10mg/kg i.p. | 16 |
| 8 n=4 | Saline | Saline | MK801 | 0 |

### Results

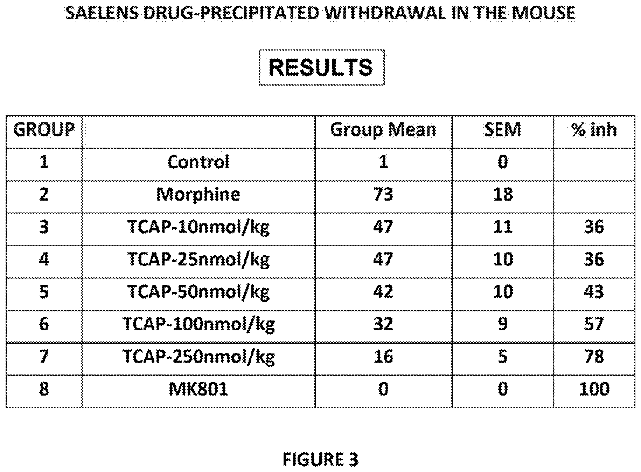

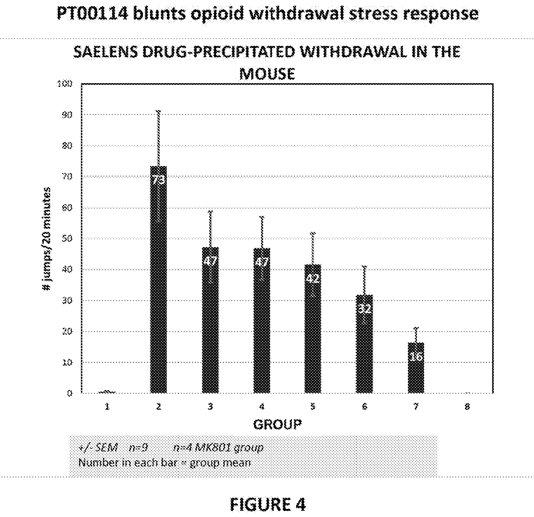

Results are illustrated in **Table 1**, the table of **Figure 3** and the bar graph of **Figure 4****,** wherein the mice administered no TCAP-1 and just morphine had the highest jump rate while those administered TCAP-1 showed reduced jump rates in a dose dependent manner.

### EXAMPLE 2: Saelens Precipitated Withdrawal Test - Single Dose TCAP Post - Morphine

### Protocol

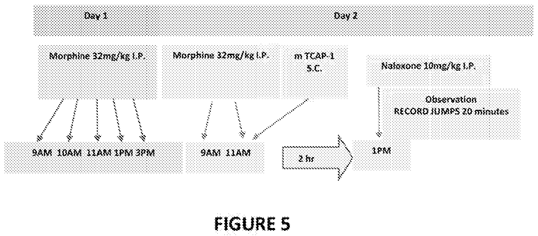

A schematic of the protocol can be seen in **Figure 5****.**

52 Male, Swiss Webster mice from Charles River Laboratory (18 - 22 g) were divided into four groups.

On Day-1, all received receive five intraperitoneal injections of Compound 1 (Morphine or Saline) at 9am, 10am, 11am, 1pm and 3pm, day 1. Group 1 (n=8) received saline solution, which Groups 2 - 4 received morphine.

On day-2, the mice received two intraperitoneal injections (9am and 11 am) of the saline (Group 1) or morphine (Groups 2 - 4) solution.

At the same time of the 11 a.m. injection, mice received subcutaneously saline solution (Groups 1 and 2), TCAP-1 at 250 nmole/kg (Group 3) or TCAP-1 at 500 nmole/kg (Group 4).

Two hours after the 11 a.m. injections, all mice received an intraperitoneal injection of Naloxone 10 mg/kg) and were immediately placed individually in an observation area and video recorded for 20 minutes. The number of jumps (lifting of all feet off the ground) was recorded for 20 minutes. The following table illustrates the study design.

**TABLE 2**

| **Group** | | **Day - 1** | | |
|---|---|---|---|---|
| | **9 a.m. , 10 a.m., 11 a.m., 1 p.m. and 3p.m. ;and** | | **Day - 2** | **Day - 2** |
| | | **Day - 2** | **11 a.m.** | **1 p.m.** |
| | **9 a.m. and 11 a.m.** | | | |
| 1 n=8 | | Saline | Saline S.C. | Naloxone 10mg/kg i.p. |
| 2 n=20 | | Morphine | Saline S.C. | Naloxone 10mg/kg i.p. |
| 3 n=12 | | 32 mg/kg i.p. | TCAP-1 250nmole/kg S.C. | Naloxone 10mg/kg i.p. |
| 4 n=12 | | | TCAP-1 500nmole/kg S.C. | Naloxone 10mg/kg i.p. |

Mouse TCAP-1 s.c. (10 mg.ml sterile water, ammonium hydroxide stock solution; Morphine 32mg/kg i.p. and Naloxone 10mg/kg i.p.

### Results

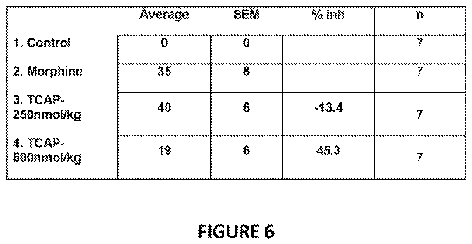

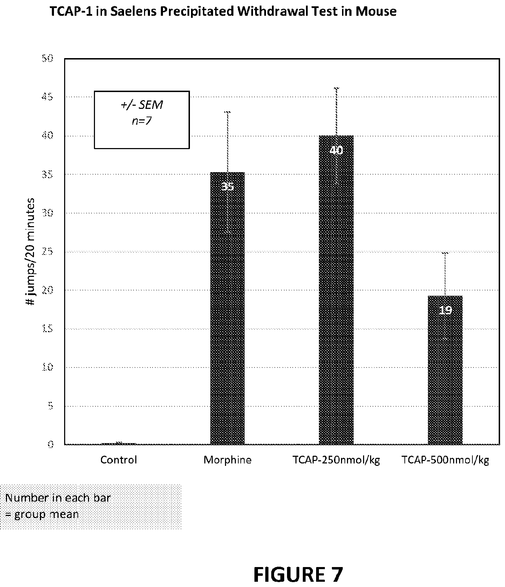

Results are illustrated in **Figures 6** **and** **7** where it can be seen that TCAP-1 significantly reduced the number of jumps in Group 4 mice.

### EXAMPLE 3: Saelens Precipitated Withdrawal Test - CRH Antagonist vs. TCAP-1

### Protocol

Forty-eight (48 Male, Swiss Webster mice from Charles River Laboratory (18-22 g) were used in Example 3.

### Protocol A

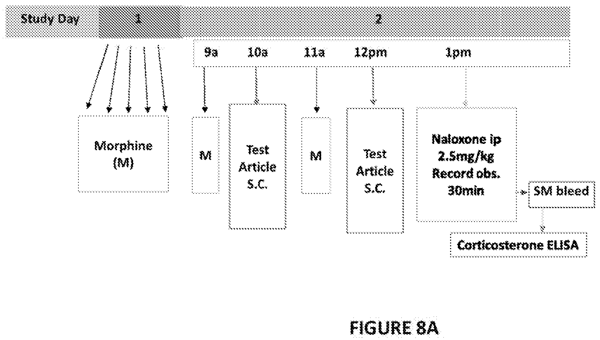

As illustrated in **Figure 8A**, 32 Male, Swiss Webster mice from Charles River Laboratory (18 - 22 g) were divided into four groups.

On day 1, all mice received five intraperitoneal injections of Compound 1 (Morphine (3.2 mg/kg) or Saline) at 9am, 10am, 11am, 1pm and 3pm. Group 1 (n=8) received saline solution, while Groups 2 - 4 received morphine.

On day 2, the mice received two intraperitoneal injections (9am and 11 am) of the saline (Group 1) or morphine (Groups 2 - 4) solution.

On the same day, at 10 a.m and 12 p.m. the mice received subcutaneously injections as follows: saline solution (Group 1), morphine (Group 2), CHR Antagonist CP154,525 20 mg/kg per time point (Group 3), or TCAP-1 at 250 nmole/kg per time point (Group 4).

Two hours after the 11 a.m. injections, at 1 p.m., all mice received an intraperitoneal ("i.p.") injection of Naloxone 2.5 mg/kg), baseline blood samples were taken and then they were immediately placed individually in an observation area and video recorded for 20 minutes. The number of jumps (lifting of all feet off the ground) were recorded for 20 minutes. A second set of blood samples were taken post-jumps (about 30 minutes after the naloxone administration.

### Results

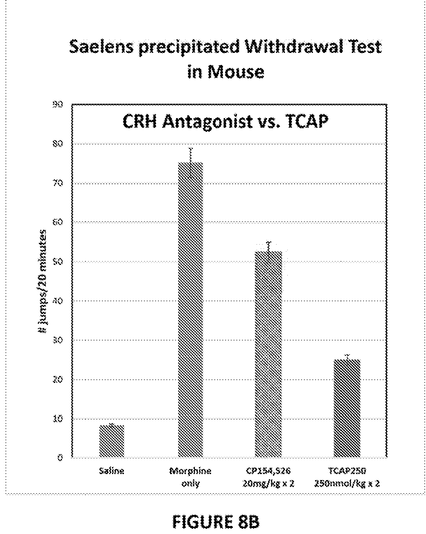

**Figure 8B** illustrates that TCAP-1 was much more effective and at lower dosages in significantly lowering the number of jumps in opioid injected mice and thus more effective in opioid withdrawal and treating opioid addictions.

### Protocol B

The same protocol as in Protocol A was followed with the exception that on day 2, there were three Group 3 mice (10 per group) which were administered: 5 mg/kg, 20 mg/kg or 50 mg/kg of the CRH antagonist CP154,526.

### Results

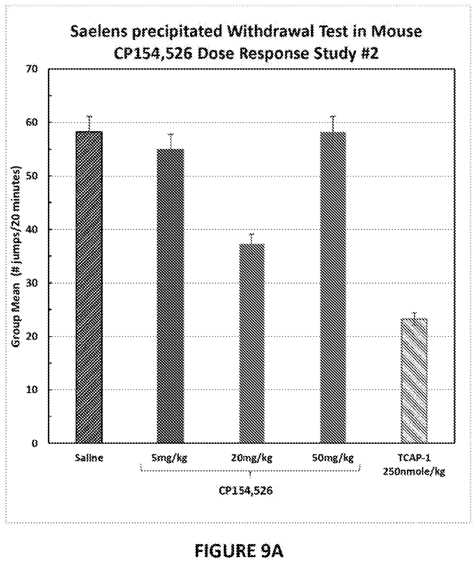

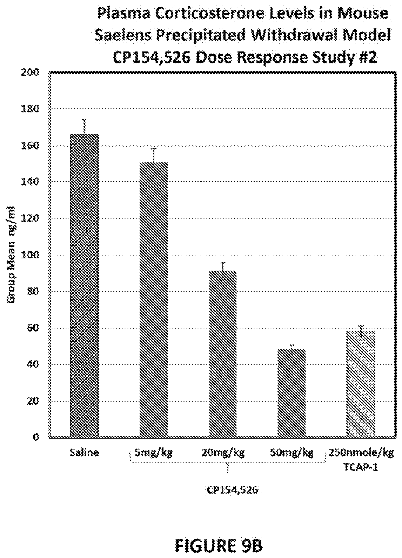

**Figures 9** **A and 9B** illustrate that TCAP-1 is more effective in reducing the number of jumps/20 minutes in the mice, and more effective in reducing plasma corticosterone levels in the mice at lower dosage levels. One would need at least 50mg/kg of CP154,526 to approach TCAP-1 250nmole/kg effect on corticosterone levels. The figures illustrate that receptors likely reached saturation at 50mg/kg CP154,526 (See **Figure 9A****),** but still had an effect (see **Figure 9B****).**

### EXAMPLE 4: Saelens Precipitated Withdrawal Test - Single Dose TCAP Post Fentanyl

### Protocol

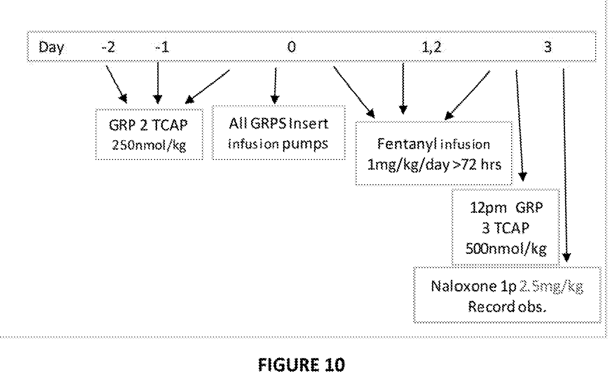

As illustrated in **Figure 10**, fentanyl instead of morphine was used as the opioid.

30 Male, Swiss Webster mice from Charles River Laboratory (18 - 22 g) were divided into three groups (n=10/group).

On days -2, -1 and day 0, all mice were administered saline (Group 1) or TCAP-1 250 nmol/kg (Groups 2 and 3) at 10 a.m. each day.

On day 0 an infusion pump was inserted into each mice was administered fentanyl by infusion for a total of 1 mg/kg /day at days 0, 1, and 2. A pump was used to get high levels of fentanyl (which normally clears quickly) in the system and to induce addiction.

At 12 p.m. on Day 3, Group 3 received a subcutaneous injection of 500 nmol/kg of TCAP-1.

At 1 p.m. on Day 3, all mice received an intraperitoneal ("i.p.") injection of Naloxone 2.5 mg/kg), and then they were immediately placed individually in an observation area and video recorded for 20 minutes. The number of jumps (lifting of all feet off the ground) were recorded for 20 minutes.

### Results

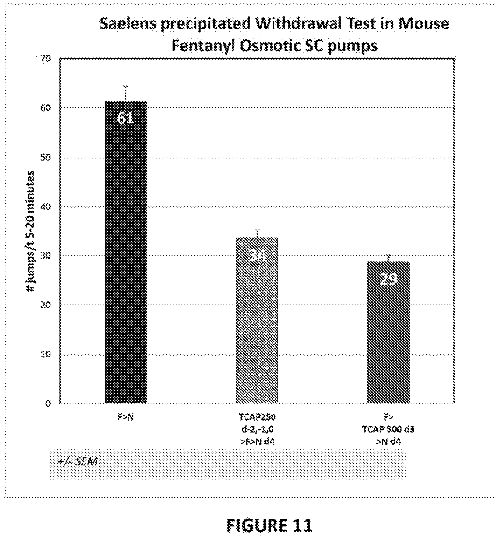

As illustrated in **Figure 11**, TCAP-1 also resulted in lower number of jumps/t 5-20 minutes in a dose dependent manner than control illustrating that TCAP-1 is effective in the treatment of opioid addiction.

## Claims

1. A teneurin c-terminal associated peptide-1 (TCAP-1 peptide) in a therapeutically effective amount, or a pharmaceutically acceptable salt or ester thereof or a pharmaceutical composition comprising the same for use in preventing and/or treating opioid addiction in a subject in need thereof wherein the amino acid sequence of said TCAP-1 peptide consists essentially of:
(i) an amino acid sequence having at least 95% identity to an amino acid sequence selected from the group consisting of SEQ ID NOs: 1, 2 or 3;
optionally wherein:
(a) the carboxy terminal end of said peptide is amidated or comprises an amidation signal sequence; and/or
(b) when the amino terminal amino acid of said peptide is a glutamine the glutamine is in the form of pyroglutamic acid.

2. The peptide or pharmaceutical composition of claim 1 for the use of claim 1 wherein the glutamine is in the form of pyroglutamic acid.

3. The peptide or pharmaceutical composition of claim 1 for the use of claim 1 wherein the TCAP-1 peptide consists of any one of SEQ ID NOs: 1, 2 or 3 which is optionally amidated at the carboxy terminal and wherein the glutamine is optionally a pyroglutamic acid at the amino terminal.

4. The peptide or pharmaceutical composition of claim 3 for the use of claim 3 wherein the TCAP-1 is amidated at the carboxy terminal.

5. The peptide or pharmaceutical composition of claim 4 for the use of claim 4 wherein the glutamine at the amino terminal is a pyroglutamic acid.

6. The peptide or pharmaceutical composition of claim 4 for the use of claim 4 wherein the TCAP-1 is SEQ ID NO.1.

7. The peptide or pharmaceutical composition of claim 4 for the use of claim 4 wherein the TCAP-1 is SEQ ID NO.2.

8. The peptide or pharmaceutical composition of any one of claims 1 to 7 for the use of any one of claims 1 to 7 wherein the subject is a human.

9. The peptide or pharmaceutical composition of any one of claims 1-8 for the use of any one of claims 1-8 wherein the opioid is selected from the group consisting of: natural, synthetic or semi-synthetic opioids.

10. The peptide or pharmaceutical composition of claim 9 for the use of claim 9 wherein the opioid is selected from one or more of the group: morphine, fentanyl, heroin, opium, oxycodone, hydrocodone, Codeine, and Methadone.

11. The peptide or pharmaceutical composition of any one of claims 1-10 for the use of any one of claims 1-10 wherein the TCAP-1 is administered before, with or after the ceasing of use of the opioid.

12. The peptide or pharmaceutical composition of claim 11 for the use of claim 11 wherein naloxone and TCAP-1 are administered together or sequentially.

## Patentansprüche

1. Teneurin-C-Terminus-assoziiertes-Peptid-1 (TCAP-1-Peptid) in einer therapeutisch wirksamen Menge oder pharmazeutisch akzeptables Salz oder pharmazeutisch akzeptabler Ester davon oder pharmazeutische Zusammensetzung, die diese umfasst, zur Verwendung bei der Vorbeugung und/oder Behandlung von Opioidabhängigkeit bei einem Individuum, das dies benötigt, wobei die Aminosäuresequenz des TCAP-1-Peptids im Wesentlichen aus Folgendem besteht:
(i) einer Aminosäuresequenz mit mindestens 95 % Identität zu einer aus der aus SEQ ID NO: 1, 2 oder 3 bestehenden Gruppe ausgewählten Aminosäuresequenz; wobei optional:
(a) das carboxyterminale Ende des Peptids amidiert ist oder eine Amidierungssignalsequenz umfasst und/oder,
(b) wenn die aminoterminale Aminosäure des Peptids ein Glutamin ist, das Glutamin in Form von Pyroglutaminsäure vorliegt.

2. Peptid oder pharmazeutische Zusammensetzung nach Anspruch 1 zur Verwendung nach Anspruch 1, wobei das Glutamin in Form von Pyroglutaminsäure vorliegt.

3. Peptid oder pharmazeutische Zusammensetzung nach Anspruch 1 zur Verwendung nach Anspruch 1, wobei das TCAP-1-Peptid aus einer der SEQ ID NO: 1, 2 oder 3 besteht, die optional am Carboxyterminus amidiert ist, und wobei das Glutamin optional eine Pyroglutaminsäure am Aminoterminus ist.

4. Peptid oder pharmazeutische Zusammensetzung nach Anspruch 3 zur Verwendung nach Anspruch 3, wobei das TCAP-1 am Carboxyterminus amidiert ist.

5. Peptid oder pharmazeutische Zusammensetzung nach Anspruch 4 zur Verwendung nach Anspruch 4, wobei das Glutamin am Aminoterminus eine Pyroglutaminsäure ist.

6. Peptid oder pharmazeutische Zusammensetzung nach Anspruch 4 zur Verwendung nach Anspruch 4, wobei das TCAP-1 SEQ ID NO: 1 ist.

7. Peptid oder pharmazeutische Zusammensetzung nach Anspruch 4 zur Verwendung nach Anspruch 4, wobei das TCAP-1 SEQ ID NO: 2 ist.

8. Peptid oder pharmazeutische Zusammensetzung nach einem der Ansprüche 1 bis 7 zur Verwendung nach einem der Ansprüche 1 bis 7, wobei das Individuum ein Mensch ist.

9. Peptid oder pharmazeutische Zusammensetzung nach einem der Ansprüche 1-8 zur Verwendung nach einem der Ansprüche 1-8, wobei das Opioid aus der aus: natürlichen, synthetischen oder semisynthetischen Opioiden bestehenden Gruppe ausgewählt ist.

10. Peptid oder pharmazeutische Zusammensetzung nach Anspruch 9 zur Verwendung nach Anspruch 9, wobei das Opioid aus einem oder mehreren aus der Gruppe mit: Morphin, Fentanyl, Heroin, Opium, Oxycodon, Hydrocodon, Codein und Methadon ausgewählt ist.

11. Peptid oder pharmazeutische Zusammensetzung nach einem der Ansprüche 1-10 zur Verwendung nach einem der Ansprüche 1-10, wobei das TCAP-1 vor, mit oder nach dem Unterlassen der Verwendung des Opioids verabreicht wird.

12. Peptid oder pharmazeutische Zusammensetzung nach Anspruch 11 zur Verwendung nach Anspruch 11, wobei Naloxon und TCAP-1 zusammen oder nacheinander verabreicht werden.

## Revendications

1. Peptide-1 associé en terminaison c de téneurines (peptide TCAP-1) en une quantité thérapeutiquement efficace, ou sel ou ester pharmaceutiquement acceptable correspondant ou composition pharmaceutique comprenant celui-ci pour une utilisation dans la prévention et/ou le traitement d'une addiction à un opioïde chez un sujet qui en a besoin, la séquence d'acides aminés dudit peptide TCAP-1 étant essentiellement constituée de :
(i) une séquence d'acides aminés ayant au moins 95 % d'identité avec une séquence d'acides aminés choisie dans le groupe constitué par les SEQ ID NO: 1, 2 ou 3 ;
éventuellement :
(a) l'extrémité terminale carboxy dudit peptide étant amidée ou comprenant une séquence de signal d'amidation ; et/ou
(b) lorsque l'acide aminé amino terminal dudit peptide est une glutamine, la glutamine étant sous la forme d'acide pyroglutamique.

2. Peptide ou composition pharmaceutique selon la revendication 1 pour l'utilisation selon la revendication 1, la glutamine étant sous la forme d'acide pyroglutamique.

3. Peptide ou composition pharmaceutique selon la revendication 1 pour l'utilisation selon la revendication 1, le peptide TCAP-1 étant constitué de l'une quelconque parmi les SEQ ID NO: 1, 2 ou 3 qui est éventuellement amidée au niveau de la terminaison carboxy et la glutamine étant éventuellement un acide pyroglutamique au niveau de la terminaison amino.

4. Peptide ou composition pharmaceutique selon la revendication 3 pour l'utilisation selon la revendication 3, le TCAP-1 étant amidé au niveau de la terminaison carboxy.

5. Peptide ou composition pharmaceutique selon la revendication 4 pour l'utilisation selon la revendication 4, la glutamine au niveau de la terminaison amino étant un acide pyroglutamique.

6. Peptide ou composition pharmaceutique selon la revendication 4 pour l'utilisation selon la revendication 4, le TCAP-1 étant la SEQ ID NO: 1.

7. Peptide ou composition pharmaceutique selon la revendication 4 pour l'utilisation selon la revendication 4, le TCAP-1 étant la SEQ ID NO: 2.

8. Peptide ou composition pharmaceutique selon l'une quelconque des revendications 1 à 7 pour l'utilisation selon l'une quelconque des revendications 1 à 7, le sujet étant un humain.

9. Peptide ou composition pharmaceutique selon l'une quelconque des revendications 1 à 8 pour l'utilisation selon l'une quelconque des revendications 1 à 8, l'opioïde étant choisi dans le groupe constitué par : des opioïdes naturels, synthétiques ou semi-synthétiques.

10. Peptide ou composition pharmaceutique selon la revendication 9 pour l'utilisation selon la revendication 9, l'opioïde étant choisi parmi l'un ou plusieurs du groupe : morphine, fentanyl, héroïne, opium, oxycodone, hydrocodone, codéine et méthadone.

11. Peptide ou composition pharmaceutique selon l'une quelconque des revendications 1 à 10 pour l'utilisation selon l'une quelconque des revendications 1 à 10, le TCAP-1 étant administré avant, conjointement à ou après l'arrêt d'utilisation de l'opioïde.

12. Peptide ou composition pharmaceutique selon la revendication 11 pour l'utilisation selon la revendication 11, de la naloxone et du TCAP-1 étant administrés ensemble ou séquentiellement.
